(19) Europäisches Patentamt
European Patent Office
Office européen des brevets

(11) EP 0 866 048 B1

(12) **EUROPEAN PATENT SPECIFICATION**

(45) Date of publication and mention of the grant of the patent:
**19.11.2003 Bulletin 2003/47**

(51) Int Cl.7: **C07C 37/62**, B01J 31/06, C08F 8/34, C07C 39/28

(21) Application number: **98301950.6**

(22) Date of filing: **16.03.1998**

(54) **Chlorination of aromatic compounds and catalystst therefor**

Chlorierung von aromatischen Verbindungen und Katalysatoren dafür

Chloration de composés aromatiques et catalyseurs à cet effet

(84) Designated Contracting States:
**AT BE CH DE DK ES FR GB IE IT LI NL PT SE**

(30) Priority: **17.03.1997 GB 9705493**

(43) Date of publication of application:
**23.09.1998 Bulletin 1998/39**

(73) Proprietor: **The University of Wales, Swansea**
**Swansea SA2 8PP (GB)**

(72) Inventors:
- **Tzimas, Michael**
  **67283 Obrigheim (DE)**
- **Smith, Keith**
  **Swansea, SA3 5DT (GB)**
- **Brown, Christopher Martin**
  **Warrington, Cheshire, WA5 5UT (GB)**
- **Payne, Keith**
  **Accrington, Lancashire, BB5 5PQ (GB)**

(74) Representative: **Linn, Samuel Jonathan et al**
**MEWBURN ELLIS**
**York House**
**23 Kingsway**
**London WC2B 6HP (GB)**

(56) References cited:
**US-A- 3 920 757**
**US-A- 3 928 293**
**US-A- 4 564 714**
**US-A- 5 594 090**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 866 048 B1

## Description

The present invention relates to the chlorination of aromatic compounds, particularly phenols, using certain sulphur-containing organic polymer compounds as catalysts. The invention also relates to such sulphur-containing organic polymer compounds per se and to processes for preparing them.

As to sulphur-containing organic polymer compounds per se, US-A-3928293 discloses solid particulate insoluble cross-linked thiohydrocarbon polymers which form a complex with boron trihydride, while US-A-5594090 discloses sulphur-containing polymer resins useful as optical materials having a high refractive index and also prepolymers from which the polymer resins are produced. These documents do not refer to the use of such polymers as catalysts in processes for chlorination of aromatic compounds.

As to such processes, US-A-4564714 discloses a process for the preparation of 2, 4, 5-trichlorphenol by selectively chlorinating 2,5-dichlorophenol under acidic conditions using specific non-polymeric, sulphur-containing catalysts.

The regioselective mono-chlorination of phenols with sulphuryl chloride has been known since 1866, when DuBois demonstrated the treatment of molten phenol with an equimolar amount of sulphuryl chloride [Z.F.Chem. 705 (1866)]. Modern analytical techniques have shown that the reaction is not as selective as was thought by DuBois, with p-chlorophenol actually being the predominantly favoured product. More recently, the catalysis of this reaction by a combination of particular divalent sulphur compounds and metal halides has been disclosed in published US Patent No. 3920757 (Watson). One of the most preferred catalysts in this document is diphenyl sulphide, in combination with $AlCl_3$, and this catalyst has been applied to a number of further chlorination reactions by sulphuryl chloride, giving a majority of para-mono-chlorinated product over the corresponding ortho-mono-chlorinated product.

This known catalyst system however has several disadvantages, particularly when intended for use on an industrial scale. For instance, product yields are not as great as may often be desired, coupled with the fact that only limited para:ortho chlorinated product ratios have hitherto been obtainable. (The para-mono-chlorinated products are generally the more useful industrially and are therefore preferred.) Also, the undesirable isomers and polychlorinated products hinder purification and can be costly to dispose of.

Another problem is that the known catalysts are difficult to separate from the reaction products mixture. Furthermore, the presence of $AlCl_3$ as a co-catalyst may be disadvantageous in that it hydrolyses on contact with water to produce acidic products which promote corrosion of metal reaction vessels and equipment. Many of these known sulphur-containing compounds also have a strong characteristic sulphurous odour, which necessitates more careful and expensive handling and equipment if health and safety hazards are to be avoided and worker-friendliness is to be optimised. Also, these known sulphur-containing catalysts are generally not re-usable, which may lead to problems of safe and environmentally friendly disposal as well as of course being detrimental to the economics of the overall process.

The present invention aims to ameliorate at least some of the above disadvantages of the prior art by providing new sulphur-containing organic polymer (or resin) compounds as catalysts, which not only give good product yields and high para-chlorinated product:ortho-chlorinated product (para:ortho) ratios, but may also be substantially odourless and may be easily recovered by filtration, hence leading to improved economics of the process when applied industrially.

In a first aspect the present invention provides a process for the chlorination of an aromatic compound of the following formula (A):

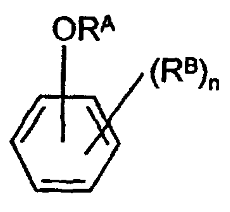

(A)

wherein $R^A$ is H or $C_1$ to $C_{12}$ alkyl, cycloalkyl, aryl, alkaryl, aralkyl or carboxyalkyl, the or each $R^B$ is selected from $C_1$-$C_4$ alkyl (especially methyl) , $C_1$-$C_4$ haloalkyl or polyhaloalkyl, e.g. $C_1$-$C_4$ perfluoroalkyl, $C_1$-$C_4$ alkoxy, $C_5$-$C_{12}$ aryl (e.g. phenyl), alkaryl or aralkyl, or halogen, and n is an integer which is 0, 1 or 2, and the or each $R^B$, if present, may independently be attached at the ortho or the meta position, preferably at the meta position, the said process comprising reacting the aromatic compound with a chlorinating agent in the presence of a sulphur-containing catalyst, optionally also in the presence of a Lewis acid co-catalyst of the formula $MX_m$, where: M is a metal or metalloid such as B, Al, Ga, In, Tl, Ge, Sn, Cd, Ni, Fe, Zn, Ti, Hg, La; X is an electronegative group such as F, Cl, Br, I, $C_1$-$C_4$ alkoxide, aryloxide e.g. phenoxide, carboxylate e.g. acetate, arenecarboxylate e.g. benzoate, substituted carboxylate e.g. trifluoroacetate, $C_1$-$C_4$ alkanesulphonate, arenesulphonate or substituted sulphonate e.g. trifluoromethanesulphonate; and m is an

integer which is preferably 1, 2, 3 or 4;
characterised in that the sulphur-containing catalyst is an organic compound according to the following formula (I) or formula (II) :

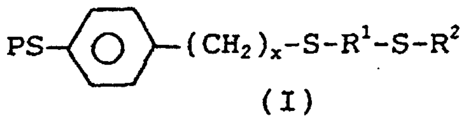

(I)

$$X^1-R^3-(S-R^4-S-R^3)_y-S-R^4-X^2$$

(II)

in which:

PS represents a polymeric backbone preferably selected from polystyrene or a polysiloxane, e.g. a polyalkylsiloxane or a polyalkylarylsiloxane;
x is an integer which is 0 or from 1 to 12;
$R^1$, $R^2$, $R^3$ and $R^4$ are each independently selected from the group consisting of: optionally substituted straight or branched chain alkyl or alkanediyl having from 1 to 20 (preferably 1 to 10) carbon atoms, and optionally substituted straight or branched chain alkaryl or aralkyl having from 5 to 20 carbon atoms;
$X^1$ and $X^2$ are each independently selected from the group consisting of halogen (preferably Cl, Br or I), thiol, dialkylsulphonium halide and $R_5$, where $R_5$ is selected from the group consisting of: optionally substituted straight or branched chain alkyl or alkanediyl having from 1 to 20 (preferably 1 to 10) carbon atoms, and optionally substituted straight or branched chain alkaryl or arallkyl having from 5 to 20 carbon atoms;
y is a number (which may be integral or non-integral) greater than zero, preferably up to about 10000, more preferably in the range from about 1 to about 1000, particularly from about 1 to about 500 and especially from about 2 to about 100;

wherein in the above definitions of $R^1$, $R^2$, $R^3$, $R^4$ and $R^5$ the optional substituents may be independently selected from the following: halogen (e.g. F, Cl), hydroxy, amino, cyano, nitro, $C_{1-4}$ alkyl, $C_{1-4}$ haloalkyl e.g. $C_{1-4}$ prefluoroalkyl, $C_{1-4}$ alkoxy and ($C_{1-4}$ alkoxy)carbonyl.

The aromatic compound of the formula (A) may be phenol or m-cresol.

In a second aspect, the present invention provides a sulphur-containing polymer compound according to the above formula (I) or according to the formula (IIa) within the formula (II), especially for use as a catalyst in the chlorination of an aromatic compound of the above formula (A) by a chlorinating agent, e.g. sulphuryl chloride.

In accordance with this second aspect, in a compound of the above formula (A), each of $R^A$, and $R^B$ and n is as defined above. Likewise, in a compound of the above formula (I), each of PS, $R^1$ and $R^2$ and x is as defined above. However, the compound of the formula (IIa) is a compound within the above formula (II) wherein each of $R^3$, $R^4$ and y is any one of the groups defined above, but each of $X^1$ and $X^2$ independently is selected specifically from halogen and dialkylsulphonium halide.

In a third aspect, the present invention provides a first process for the preparation of a sulphur-containing polymer compound of formula I according to the second aspect of the invention, this first process comprising reacting a compound of the formula

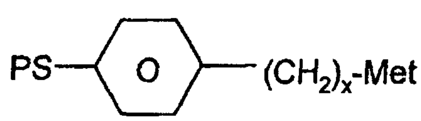

in which PS and x are as defined above and Met is an electropositive metal preferably selected from Li, Na, K, Mg or Zn,

with a disulphide compound of the formula

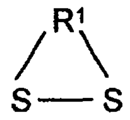

and then a compound of formula L-$R^2$

in which $R^1$ and $R^2$ are as defined above and L is a leaving group preferably selected from the group consisting of halide (e.g. $Cl^-$, $Br^-$), carboxylate and sulphonate.

In a fourth aspect the present invention provides a second process for the preparation of a sulphur-containing polymer compound of formula I according to the second aspect of the invention, this second process comprising reacting a compound of the formula

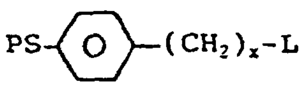

in which PS and x are as defined above and L is a leaving group preferably selected from the group consisting of halide (e.g. $Cl^-$, $Br^-$), carboxylate and sulphonate,

with a sulphur-containing nucleophile of the formula

$$R^2\text{-S-}R^1\text{-S-Met}$$

in which $R^1$, $R^2$ and Met are as defined above.

In a fifth aspect, the present invention provides a process for the preparation of a sulphur-containing polymer compound of formula IIa according to the second aspect of the invention, the process comprising reacting together a compound of the formula

$$\text{MetS-}R^4\text{-SMet}$$

in which $R^4$ and Met are as defined above

and a compound of the formula

$$X^1\text{-}R^3\text{-}X^2$$

in which $X^1$, and $X^2$ are each independently selected from halogen and dialkylsulphonium and $R^3$ is as defined above.

Preferred embodiments and features of the synthetic processes of the above third, fourth and fifth aspects of the invention are described in detail hereinbelow.

In a sixth aspect, the present invention provides the use of a sulphur-containing polymer compound of the second aspect of the invention as a catalyst in the chlorination of an aromatic compound of formula (A) above by a chlorinating agent, e.g. sulphuryl chloride.

The above primary aspects of the invention, and preferred features and embodiments thereof, will now be described in further detail.

The chlorinating agent and reaction

Conditions of the chlorination process

In the process according to the first aspect of the invention the chlorinating agent is suitably sulphuryl chloride ($SO_2Cl_2$). Other known chlorinating agents however may be suitable, e.g. chlorine gas. The process using sulphuryl

chloride with the sulphur-containing polymer or resin catalyst, and with the optional presence of the Lewis acid co-catalyst, is preferably carried out in heterogeneous liquid/solid phase, without the presence of a solvent. However, a solvent may be used if desired or necessary. Suitable solvents include alkanes such as n-hexane, THF, diethyl ether, halogenated hydrocarbons such as perchloro-ethylene or carbon tetrachloride, chloroform or dichloromethane, petrol ether, alcohols such as ethanol or methanol, water, pyridine, dimethylformamide, dimethylsulphoxide or mixtures of any of these. If a solvent is used, the temperature at which the reaction is carried out is generally lower than when there is no solvent present e.g. if phenol is the aromatic compound to be chlorinated, then in the absence of solvent the reaction mixture may solidify at a temperature less than 35°C, which sets a practical lower limit on the possible temperature for the reaction.

For maximum selectivity, it is preferable that the temperature used is as low as possible, e.g. even below 35°C, more preferably below about 20 or 25°C, even possibly down to about 0°C, but the reaction may however still work, in certain embodiments, at temperatures of 85°C or more. Often the reaction will be carried out approximately at or near room temperature, e.g. in the region of about 15 to about 30°C, in order to minimise the external heating or cooling that is required.

The amount of sulphur-containing polymer/resin catalyst present in the reaction mixture may vary from about 0.01g to about 5g for each 100mmol of the aromatic compound present in the reaction mixture. More preferably the amount of catalyst present is from about 0.1g to about 2.0g for each 100mmol of the aromatic compound, and within this preferred range lower amounts are more preferred.

The amount of sulphuryl chloride ($SO_2Cl_2$) (or other chlorinating agent) present in the reaction mixture may also vary, e.g. depending on the amount of aromatic compound to be reacted or whether solvent is used. Preferably the $SO_2Cl_2$ is provided in small excess, with respect to the amount of the aromatic compound, e.g. up to about 100 mol% excess. If desired or necessary, it may be possible for the $SO_2Cl_2$ to be present in molar deficit, e.g. up to about 20mol% deficit. The most preferred amount of $SO_2Cl_2$ present in the reaction mixture is approximately at a 2 to 20mol% excess over the amount of aromatic compound.

The chlorination reaction may be carried out by the slow addition of the $SO_2Cl_2$ to a reaction mixture consisting of or containing the aromatic compound, the polymer catalyst, the Lewis acid co-catalyst (if present), and the solvent (if present). Following the addition a period of stirring is generally used to ensure that the reaction is substantially complete, whilst minimising cost.

The optional presence of the above defined Lewis acid co-catalyst in the reaction mixture may further increase the para:ortho product ratio and also the yield of the mono-chlorinated product. However, in certain industrial processes the presence of such a Lewis acid may cause a problem as regards corrosion of equipment hardware and in its separation from effluent streams, so in certain practical embodiments of the process of the invention the use of a Lewis acid co-catalyst may be less desirable. A metal halide such as $AlCl_3$ is often the most selective and thus preferable Lewis acid when used, although others may perform reasonably well. Both $FeCl_3$ and $ZnCl_2$ for instance have fewer practical and environmental disadvantages than $AlCl_3$ for large scale use. When used the amount of Lewis acid in the reaction mixture (in relation to the amount of the aromatic compound) may vary between approximately 0.1 and 15 mol%, with the preferred amount being about at a 1 to 40 mol% excess over the amount of resin catalyst used.

The scale of the chlorination process may vary, one advantage of the invention being that having a larger scale process may not deleteriously affect the para:ortho product ratio or the overall yield of the desired product from the reaction.

The Sulphur-containing Resin Catalysts

The sulphur-containing resin catalysts of general formulae I or II above may be synthesized by the respective processes defined generally hereinabove as the third, fourth and fifth aspects of the invention.

In preferred embodiments, resin catalysts of general formula (I) may be synthesised by either (A) lithiating a halogenated polystyrene or polysiloxane resin, reacting the product with a cyclic disulphide and then an electrophile, or (B) first opening up a cyclic disulphide with an organolithium reagent and then reacting this product with a halogenated polystyrene or polysiloxane resin. These two methods can be represented by the following equations:

Method A

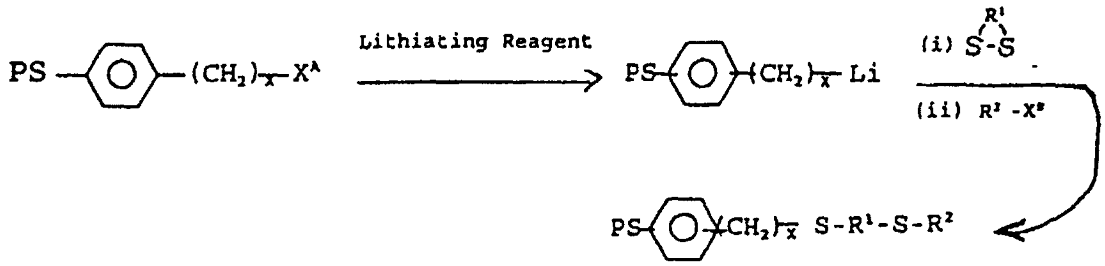


Method B

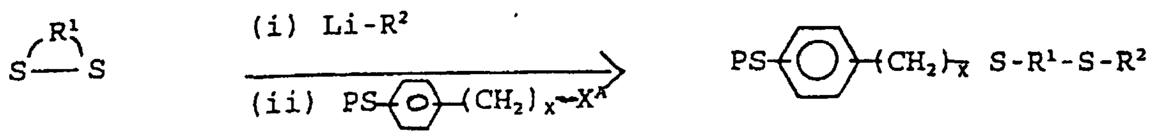


in which $X^A$ and $X^B$ are each independently selected from Cl, Br, or I. Such halogenated polystyrene or polysiloxane resins may be commercially available, for example as bromopolystyrene (BrPS) and Merrifield Resin (MR), or alternatively may be synthesized by methods well known in the art and readily available to the skilled person. The Merrifield Resin is of the formula:

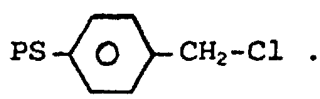

The cyclic disulphides used in the above two methods may be readily synthesised by oxidative cyclisation of the readily available dithiols according to well known literature procedures, e.g. using as reagents iodine and triethylamine in trichloromethane, which reaction can be represented by the following equation:

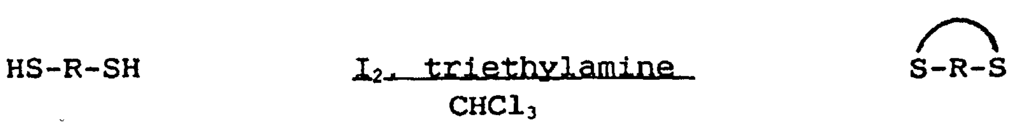


In sulphur-containing polymers of general formula (I) $R^1$ and $R^2$ are preferably each independently selected from optionally substituted straight or branched chain alkyl or alkanediyl having from 1 to 10 carbon atoms. Preferably both groups $R^1$ and $R^2$ are unsubstituted and/or unbranched. It is further preferred that $R^1$ and $R^2$ each independently have from 4 to 10 carbon atoms, most preferably from 4 to 6 carbon atoms.

In preferred embodiments, resin catalysts of general formula (II) above may be synthesised from the functionalised monomers HS-$R^4$-SH and $X^1$-$R^3$-$X^2$. This synthesis may for example be carried out by lithiating the dithiol with an organo-lithium reagent, then reacting the product with the dihalo compound, e.g. at room temperature in a suitable solvent such as e.g. THF, to yield the overall polymeric compound with general formula (II). If $X^1$ and/or $X^2$ is the group $R^5$ in formula (II), then a further reactive step is preferably carried out, namely reacting the intermediate

product with a halo-compound, Hal-$R^5$, e.g. at room temperature.

In polymer compounds of general formula (II) both of $X^1$ and $X^2$ are preferably Br. More preferably, $R^3$, $R^4$ and $R^5$ (if present) are each independently unbranched and/or unsubstituted alkyl groups having from 1 to 10 carbon atoms. Most preferred is when $R^3$, $R^4$ and $R^5$ (if present) are each independently a $C_6$-$C_8$ alkyl group.

EXAMPLES

Preferred features and embodiments of the present invention in its various aspects will now be illustrated in detail by way of the following examples.

Comparative Examples 1 to 3

In the following Comparative Examples 1 to 3, the chlorination reaction:

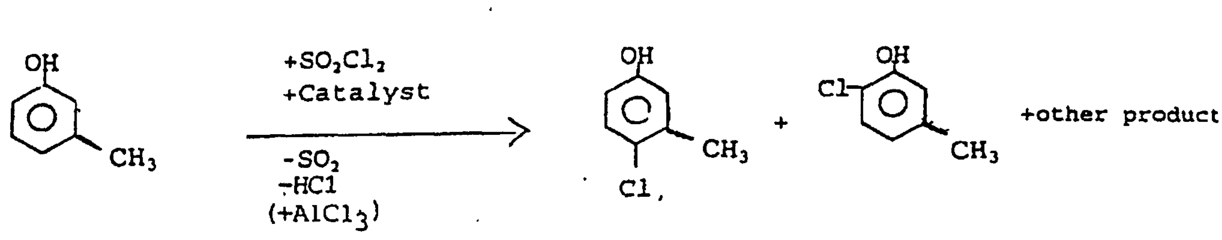


is carried out as follows.

A clean and dry 250ml two necked, round bottom flask is flushed with $N_2$ for a few seconds, to prevent oxidation of meta-cresol and hydrolysis of $AlCl_3$, if present. 100mmol (10.81g) of meta-cresol is then placed in the flask along with 2.69mmol of the catalyst used, 3.75mmol $AlCl_3$ if required and a magnetic follower. The flask is fitted with a stopper and a pressure equalising dropping funnel. 110mmol (8.8ml) of freshly distilled sulphuryl chloride (see below) is placed in the dropping funnel and the funnel fitted with a $CaCl_2$ drying tube. The reaction mixture is stirred while the $SO_2Cl_2$ is added at a rate of approximately one drop every two seconds. The addition rate is checked from time to time and the addition finished within two hours. Stirring is then maintained for another 2 hours.

The reaction is quenched with 30ml of distilled water and stirred for 30 minutes to hydrolyse residual $SO_2Cl_2$. The two phases are transferred into a separating funnel and the flask rinsed with distilled water and diethyl ether. The mixture is extracted twice with 30ml of diethyl ether and the combined ether layers are washed with 10ml of distilled water. The organic phase is then dried over $MgSO_4$ and filtered. The organic phase is evaporated under vacuum (18mbar) at 50°C. The product is then subjected to elemental analysis and analysed by gas chromatography, as described further below.

The freshly distilled sulphuryl chloride mentioned above is prepared by placing sulphuryl chloride in a double-necked, round-bottom flask with a few anti-bumping granules. The flask is set up for distillation and fitted with a nitrogen inlet and a water cooled condenser leading to a three way receiver and a nitrogen outlet. The gas flows out through a scrubber. The system is flushed with nitrogen and then the gas flow is halted. The flask is heated and the fraction boiling at 67-69°C is collected as sulphuryl chloride. This colourless liquid is stored under nitrogen.

In order to analyse the purified reaction products, a precisely known amount of the stored sample (approximately 500mg) and 100mg tetradecane as internal standard are weighed into a flask and diluted with 25ml dichloromethane. 1μl of the resulting solution is injected onto a gas chromatograph for analysis.

The gas chromatography system used involves a Philips PU 4400 instrument with a PU 4920 data station providing control, data storage and manipulation for the gas chromatograph. The conditions set for analysis are:

| | |
|---|---|
| column | 15m carbowax megabore, ID 0.54mm, 1.2μm film thickness |
| carrier gas | 5ml/min helium |
| make up gas | 25ml.min nitrogen |
| injector temperature | 300°C |
| detector temperature | 300°C (F.I.D.) |
| injection technique | splitless |

(continued)

| | |
|---|---|
| initial time | 2 minutes |
| column start temperature | 35°C |
| ramp rate | 20°C/min |
| upper temperature | 240°C |

The trace produced by the gas chromatograph is converted into mol% of product present in the purified reaction products using standard calculations as are well known and commonly applied in the art.

Synthesis Example 1

This example illustrates the synthesis of two sulphur-containing resin compounds of general formula (I) above (Compounds 41 and 42).

Compound 41: Bromopolystyrene (4mmol Br per gram resin, 200-400 mesh) is first washed with THF (3 x 25ml), diethyl ether (3 x 25ml), THF/$H_2O$ (2/1, 3 x 25ml), benzene (3 x 25ml), methanol (3 x 25ml) and then dried at 65°C under reduced pressure (2mbar). 0.5g, which contains approximately 2mmol of bromine, is placed in a dry round bottom flask which is then fitted with a septum and flushed with $N_2$. Dry benzene (10ml) is added via a dry syringe. The mixture is stirred for 30 minutes and then heated to 65°C. Butyllithium (1.7ml, 12mmol, 7M) is added via a dry syringe, and the mixture is stirred for another 120 minutes. The resin is washed with dry benzene (2 x 10ml). 1,2-dithiane (0.24g, 2mmol) is dissolved in dry benzene (10ml) and added to the resin. The reaction mixture is stirred for 60 minutes and then the resin is washed with benzene (2 x 10ml). 1-Bromobutane (0.55g, 4mmol) is added to the resin and stirred for 12 hours at room temperature. The resin is collected by filtration, washed with THF (3 x 25ml), diethyl ether (3 x 25ml), THF/$H_2O$ (2/1, 3 x 25ml), benzene (3 x 25ml), methanol (3 x 25ml) and dried at 65°C under reduced pressure (2mbar). The modified resin (1.228g) is isolated with a yield of lithiation of 41 mol% and an overall yield of modification of 40 mol%. The modified resin contains approximately 1.4mmol sulphur-containing functional groups per gram of resin. Elemental analysis after lithiation and then treatment with water finds C, 72.44%;H, 5.79%. After modification elemental analysis finds C, 68.46%; H, 6.89%. $[(C_8H_8)_{2.55}(C_8H_7Br)_{2.37}(C_{16}H_{24}S_2)_{1.65}]_n$ requires C, 68.16%; H, 6.67%; Br, 16.17%; S, 9.00%.

Compound **42**: Bromopolystyrene is first prepared as for the synthesis of Compound **41** above. 0.5g, which contains approximately 2mmol of bromine, is placed in a dry round bottom flask which is then fitted with a septum and flushed with $N_2$. Dry benzene (10ml) is added via a dry syringe, the mixture stirred for 30 minutes and then heated to 65°C. Butyllithium (1ml, 10mmol, 10M) is added via a dry syringe, and the mixture is stirred for another 180 minutes. The resin is washed with dry benzene (2 x 10ml). 1,2-Dithiane (0.5g, 4.1mmol) is dissolved in dry benzene (10ml) and added to the resin. The reaction mixture is stirred for 30 minutes and then the resin is washed with benzene (2 x 10ml). 1-Bromobutane (0.55g, 4mmol) is added to the resin and stirred for 12 hours at room temperature. The resin is collected by filtration, washed with THF (3 x 25ml), diethyl ether (3 x 25ml), THF/$H_2O$ (2/1, 3 x 25ml), benzene (3 x 25ml), methanol (3 x 25ml) and dried at 65°C under reduced pressure (2mbar). The modified resin is isolated with an overall yield of modification of 71 mol%. The modified resin contains approximately 2.3mmol sulphur- containing functional groups per gram of resin. Elemental analysis after lithiation and treatment with water finds C, 84.82%;H, 8.12%. After modification elemental analysis finds C, 73.16%; H, 8.36%. $[(C_8H_8)_{3.08}(C_8H_7Br)_{0.66}(C_{16}H_{24}S_2)_{2.84}]_n$ requires C, 73.16; H, 7.94%.

Comparative Example 1

Table 1 below compares the catalytic effect of an unmodified bromopolystyrene resin and a modified bromopolystyrene resin catalyst synthesised as above, (Compound **42**) in the absence of any Lewis acid. The effect of the unmodified bromopolystyrene resin is comparable to that achieved when no catalyst is present, when the para:ortho ratio is approximately 8.7 and the mol% of parachlorometa-cresol is about 81. The modified bromopolystyrene resin performs poorly and decreases the para- selectivity of the reaction. Without being bound by any particular theory, it is believed that this may be caused by the presence of local hotspots where the concentration of $SO_2Cl_2$ is high, caused by the high viscosity of the reaction mixture. Lowering the concentration may improve the selectivity and overall yield (see Comparative Example 2 below).

**Table 1: Chlorination of *meta*-cresol in the presence of modified bromopolystyrene resins[a]**

| catalyst | amount catalyst g/100 mmol MC | MC mol%[b] | OCMC mol%[b] | PCMC mol%[b] | p/o ratio | mass balance %[c] |
|---|---|---|---|---|---|---|
| 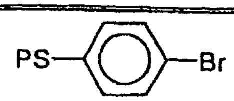 | 1.0 | 10.6 | 9.6 | 69.2 | 7.2 | 89.4 |
| 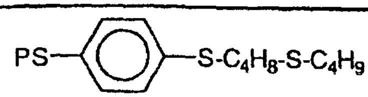 | 1.2 | 10.4 | 13.0 | 71.2 | 5.5 | 94.6 |
| 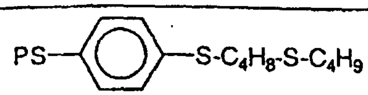 reused | 1.17 | 8.2 | 12.6 | 66.6 | 5.3 | 87.4 |

Notes

a) 100 mmol MC, 110 mmol $SO_2Cl_2$, 4h, room temperature; b) expressed as the absolute yield of the compounds listed, as determined by quantitative GC; c) sum of MC+OCMC+PCMC; a water ether mixture was used to wash the resin and this caused difficulties with separation and consequent loss of material.

Synthesis Example 2

This example illustrates the synthesis of two further sulphur-containing polymer compounds of general formula (I) (Compounds **43** and **44**).

Compound **43**: Merrifield resin (1.7mmol Cl per gram resin) is prepared for the synthesis by washing it with THF (3 x 25ml), diethyl ether (3 x 25ml), THF/$H_2O$ (2/1, 3 x 25ml), benzene (3 x 25ml), methanol (3 x 25ml) and then dried at 65°C under reduced pressure (2mbar). 1,2-Dithiane (1.5g, 12.6mmol) is placed in a dry round bottom flask which is then fitted with a septum and flushed with $N_2$. Dry THF (30ml) is added via a dry syringe, and the mixture cooled to -78°C in a cardice/acetone bath. Butyllithium (5.9ml, 12.4mmol, 2.1M) is added via a dry syringe over 20 minutes, and the mixture is stirred at -78°C for another 20 minutes. The reaction is allowed to warm up to ambient temperature. The previously prepared Merrifield resin (3g, 5.1mmol Cl) is added rapidly with nitrogen flushing to the organolithium compound and the mixture stirred overnight at room temperature. The resin is collected by filtration, washed with THF (3 x 25ml), diethyl ether (3 x 25ml), THF/$H_2O$ (2/1, 3 x 25ml), benzene (3 x 25ml) and methanol (3 x 25ml) and dried at 65°C under reduced pressure (2mbar). Compound **43** is isolated with a yield of 93 mol% and 100% conversion of the functional group. Elemental analysis finds C, 82.89%; H, 8.58%. $[(C_8H_8)_{7.12}(C_{17}H_{26}S_2)_{1.70}]_n$ requires C, 83.07%; H, 8.16%.

Compound **44**: 1,2-Dithiacyclooctane (0.74g, 5mmol) is placed in a dry round bottom flask which is then fitted with a septum and flushed with $N_2$. Dry THF (30ml) is added via a dry syringe, and the mixture cooled to -78°C in a cardice/acetone bath. Butyllithium (1.9ml, 4.75mmol, 2.5M) is added via a dry syringe over 20 minutes, and the mixture is stirred at -78°C for another 20 minutes. The reaction is allowed to warm up to ambient temperature. The previously prepared Merrifield resin, see Compound 43, (1g, 1.7mmol Cl) is added rapidly with nitrogen flushing to the organolithium compound and the mixture stirred overnight at room temperature. The resin is collected by filtration, washed with THF (3 x 25ml), diethyl ether (3 x 25ml), THF/$H_2O$ (2/1, 3 x 25ml), benzene (3 x 25ml) and methanol (3 x 25ml) and dried at 65°C under reduced pressure (2mbar). Compound 44 is isolated with a yield of 95 mol% and 100% conversion of the functional group. Elemental analysis finds C, 82.98%; H, 8.90%. $[(C_8H_8)_{7.12}(C_{17}H_{26}S_2)_{1.70}]_n$ requires C, 83.07%; H, 8.16%.

Comparative Example 2

Table 2 below compares the catalytic effect of an unmodified Merrifield resin and modified Merrifield resin compound, synthesised as the above Compounds **43** and **44**, in the absence or presence of $AlCl_3$. The effect of the unmodified Merrifield resin, which is washed before use to remove low molecular weight impurities, is comparable to that achieved when no catalyst is present (the baseline results), when the para:ortho ratio is approximately 8.7 and the mol% of parachlorometa-cresol is about 81.

The second entry in Table 2 shows the influence of Compound 43 on the chlorination of meta-cresol in the absence of $AlCl_3$. The results are worse than the baseline results, but reducing the amount of catalyst used, and using $AlCl_3$ (as shown in entry 3 in the Table), produces dramatically improved results.

The last three entries in Table 2 below utilise Compound **44,** in which $R_1$ of the general formula I is a $C_6$ alkyl group. The selectivity achieved in entry 4 in the Table is similar to the baseline result. Carrying out the reaction in the presence of $AlCl_3$ (see entries 5 and 6 in Table 2) increases the para:ortho product ratio, and the amount of residual meta-cresol is small.

Table 2: Chlorination of *meta*-cresol in the presence of modified Merrifield resins[a]

| catalyst | amount catalyst g/100 mmol MC | $AlCl_3$ mmol | MC mol%[b] | OCMC mol%[b] | PCMC mol%[b] | *p/o* ratio | mass balance %[c] |
|---|---|---|---|---|---|---|---|
| 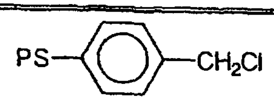 | 1.0 | - | 11.2 | 8.4 | 61.6 | 7.4 | 81.2 |
| 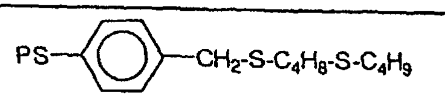 | 2.0 | - | 12.0 | 15.6 | 67.8 | 4.3 | 95.8 |
| 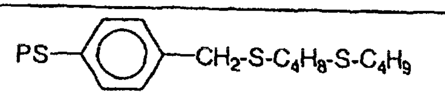 | 0.5 | 3.75 | 7.6 | 6.4 | 81.0 | 12.7 | 95.0 |
| 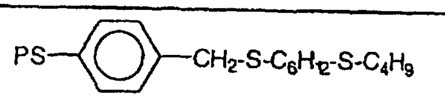 | 0.5 | - | 8.6 | 10.4 | 76.6 | 7.3 | 95.6 |
| 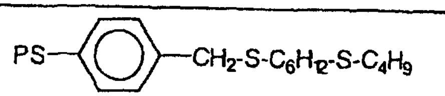 | 0.5 | 3.75 | 0.4 | 5.2 | 90.4 | 17.4 | 96.0 |

a) 100 mmol MC, 110 mmol $SO_2Cl_2$, 4h, room temperature; b) expressed as the absolute yield of the compounds listed, as determined by quantitative GC; c) sum of MC+OCMC+PCMC; a water ether mixture was used to wash the resin and this caused difficulties with separation and consequent loss of material.

Synthesis Example 3

This example illustrates the synthesis of a sulphur-containing polymer of general formula (II).

Compound **45**: 1,6-Hexanedithiol (2.26g, 15mmol) is placed in a 100ml dry round bottom flask which is then fitted with a septum and flushed with $N_2$. Dry THF (20ml) is added via a dry syringe and the mixture cooled to -78°C in a cardice/acetone bath. Butyllithium (12ml, 30mmol, 2.5M) is added via a dry syringe over 20 minutes and the mixture stirred at -78°C for another 30 minutes. The reaction is then allowed to warm up to room temperature. 1,8-Dibromooctane (3.76g, 13.5mmol) is added via a syringe and the reaction stirred at room temperature for 60 minutes. 1-Bromobutane (0.4g, 3mmol) may be added to the reaction mixture and stirring continued for 18 hours. The resin is collected by filtration, and washed with $H_2O$ (3 x 25ml), THF (3 x 25ml), hexane (3x25ml), methanol (3 x 25ml), and diethyl ether (3 x 25ml), and dried at 65°C under reduced pressure (1mbar). The polythia-alkane is isolated with a yield of 50mol% (1.952g). Elemental analysis finds C, 60.85%; H, 11.19%; S, 21.16%; Br, 7.30%. $Br-S-C_8H_{16}-S-C_6H_{12}-(S-C_6H_{12}-S-C_8H_{16})_{6.8}-Br$ requires C, 60.78%; H, 10.13%; S, 21.67%; Br 7.42%.

Comparative Example 3

Table 3 below illustrates the catalytic effect of the polythia-alkane Compound **45**, synthesised in Synthesis Example 3 above, on the chlorination reaction of meta-cresol with sulphuryl chloride. The first and second entries in the Table show the results achieved, using one gram of Compound **45** as catalyst. The selectivity obtained in the absence of $AlCl_3$ is similar to the baseline result, which is a para:ortho ratio of approximately 8.7. The addition of $AlCl_3$ increases the para:ortho product ratio to approximately 17. Reducing the amount of catalyst present in the reaction mixture to 0.2g increases the ratio to 26.6, which is a highly desirable result.

Table 3: Chlorination of *meta*-cresol in the presence of polythiaalkanes[a]

| catalyst | amount catalyst g/100 mmol MC | $AlCl_3$ mmol | MC mol% [b] | OCMC mol% [b] | PCMC mol% [b] | p/o ratio | mass balance % [c] |
|---|---|---|---|---|---|---|---|
| $Br-C_8H_{16}-S-C_6H_{12}-[S-C_6H_{12}-S-C_8H_{16}]_{6-8}-Br$ | 1.0 | - | 6.4 | 9.4 | 74.2 | 7.9 | 90.0 |
| $Br-C_8H_{16}-S-C_6H_{12}-[S-C_6H_{12}-S-C_8H_{16}]_{6-8}-Br$ | 1.0 | 3.75 | 0.4 | 4.8 | 79.8 | 16.6 | 85.0 |
| $Br-C_8H_{16}-S-C_6H_{12}-[S-C_6H_{12}-S-C_8H_{16}]_{6-8}-Br$ | 0.2 | 3.75 | 0.6 | 3.2 | 84.2 | 26.3 | 88.0 |

a) 100 mmol MC, 110 mmol $SO_2Cl_2$, 4h, room temperature; b) expressed as the absolute yield of the compounds listed, as determined by quantitative GC; c) sum of MC+OCMC+PCMC; a water ether mixture was used to wash the resin and this caused difficulties with separation and consequent loss of material.

## Claims

1. A process for the chlorination of an aromatic compound of the following formula (A):

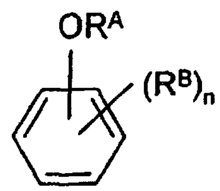

(A)

wherein $R^A$ is H or $C_1$-$C_{12}$ alkyl, cycloalkyl, aryl, alkaryl, aralkyl or carboxyalkyl, the or each $R^B$ is selected from $C_1$-$C_4$ alkyl, $C_1$-$C_4$ haloalkyl or polyhaloalkyl, $C_1$-$C_4$ alkoxy, $C_5$-$C_{12}$ aryl, alkaryl or aralkyl, or halogen, and n is an integer which is 0, 1 or 2, and the or each $R^B$, if present, may independently be attached at the ortho or the meta position, the said process comprising reacting the aromatic compound with a chlorinating agent in the presence of a sulphur-containing catalyst, optionally also in the presence of a Lewis acid co-catalyst of the formula $MX_m$, where: M is a metal or metalloid; X is an electronegative group; and m is an integer;
**characterised in that** the sulphur-containing catalyst is an organic compound according to the following formula (I) or formula (II):

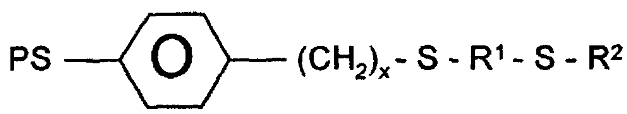

(I)

$$X^1 - R^3 - (S - R^4 - S - R^3)_y - S - R^4 - X^2 \quad \text{(II)}$$

in which:

PS represents a polymeric backbone;
x is an integer which is 0 or from 1 to 12;
$R^1$, $R^2$, $R^3$ and $R^4$ are each independently selected from the group consisting of: optionally substituted straight or branched chain alkyl or alkanediyl having from 1 to 20 carbon atoms, and optionally substituted straight or branched chain alkaryl or aralkyl having from 5 to 20 carbon atoms;
$X^1$ and $X^2$ are each independently selected from the group consisting of halogen, thiol, dialkylsulphonium halide and
$R_5$, where $R_5$ is selected from the group consisting of: optionally substituted straight or branched chain alkyl or alkanediyl having from 1 to 20 carbon atoms, and optionally substituted straight or branched chain alkaryl or aralkyl having from 5 to 20 carbon atoms;
y is a number (which may be integral or non-integral) greater than zero;

wherein in the above definitions of $R^1$, $R^2$, $R^3$, $R^4$ and $R^5$ the optional substituents may be independently selected from the following: halogen, hydroxy, amino, cyano, nitro, $C_1$-$C_4$ alkyl, $C_1$-$C_4$ haloalkyl, $C_1$-$C_4$ alkoxy and ($C_1$-$C_4$ alkoxy)carbonyl.

2. A process according to claim 1, wherein the aromatic compound is phenol.

3. A process according to claim 1, wherein the aromatic compound is meta-cresol.

4. A process according to claim 1 or claim 2, wherein the chlorinating agent is sulphuryl chloride.

5. A process according to any preceding claim, wherein the chlorination reaction is carried out in heterogeneous liquid/solid phase, without the presence of added solvent.

6. A process according to any preceding claim, wherein the chlorination reaction is carried out at a temperature of below 35°C.

7. A process according to any preceding claim, wherein the amount of sulphur-containing polymer/resin catalyst present in the reaction mixture is from 0.01g to 5g for each 100mmol of the aromatic compound present in the reaction mixture.

8. A sulphur-containing polymer compound of the formula:

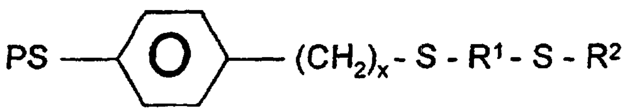

(I)

in which:

PS represents a polymeric backbone;
x is an integer which is 0 or from 1 to 12;
$R^1$ and $R^2$ are each independently selected from the group consisting of: optionally substituted straight or branched chain alkyl or alkanediyl having from 1 to 20 carbon atoms, and optionally substituted straight or branched chain alkaryl or aralkyl having from 5 to 20 carbon atoms;

wherein in the above definitions of $R^1$ and $R^2$ the optional substituents may be independently selected from the following: halogen, hydroxy, amino, cyano, nitro, $C_1$-$C_4$ alkyl, $C_1$-$C_4$ haloalkyl, $C_1$-$C_4$ alkoxy and ($C_1$-$C_4$ alkoxy) carbonyl.

9. A compound according to claim 8, wherein $R^1$ and $R^2$ are each independently selected from optionally substituted straight or branched chain alkyl or alkanediyl having from 1 to 10 carbon atoms.

10. A sulphur-containing compound of the formula

$$X^1\text{-}R^3\text{-}(S\text{-}R^4\text{-}S\text{-}R^3)_y\text{-}S\text{-}R^4\text{-}X^2 \quad \text{(IIa)}$$

in which:

$R^3$ and $R^4$ are each independently selected from the group consisting of: optionally substituted straight or branched chain alkyl or alkanediyl having from 1 to 20 carbon atoms, and optionally substituted straight or branched chain alkaryl or aralkyl having from 5 to 20 carbon atoms;
$X^1$ and $X^2$ are each independently selected from the group consisting of halogen and dialkylsulphonium halide;
y is a number (which may be integral or non-integral) greater than zero;

wherein in the above definitions of $R^3$ and $R^4$ the optional substituents may be independently selected from the following: halogen, hydroxy, amino, cyano, nitro, $C_1$-$C_4$ alkyl, $C_1$-$C_4$ haloalkyl, $C_1$-$C_4$ alkoxy and ($C_1$-$C_4$ alkoxy) carbonyl.

11. A compound according to claim 10, which is obtainable by reacting a compound of the formula $HS\text{-}R^4\text{-}SH$ with an organo-lithium reagent to form a compound of the formula $LiS\text{-}R^4\text{-}SLi$, then reacting the product with a compound of the formula $X^1\text{-}R^3\text{-}X^2$ in which $X^1$ and $X^2$ are each independently selected from a halogen and each of $R^3$ and $R^4$ is as defined in claim 10.

**12.** A compound according to claim 10, wherein both of $X^1$ and $X^2$ are halogen.

**13.** A compound according to claim 12, wherein both of $X^1$ and $X^2$ are Br.

**14.** A compound according to any one of claims 10 to 13, wherein $R^3$ and $R^4$ are each independently a $C_6$-$C_8$ alkyl group.

**15.** A process for the preparation of a sulphur-containing polymer compound of the formula (I), as defined in claim 8, the process comprising reacting a compound of the formula

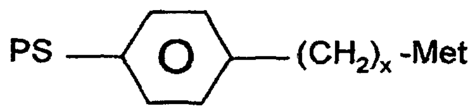

in which PS and x are as defined in claim 8 and Met is an electropositive metal,
with a disulphide compound of the formula

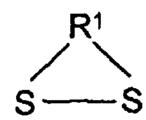

and then a compound of formula L-$R^2$
in which $R^1$ and $R^2$ are as defined in claim 8 and L is a leaving group.

**16.** A process for the preparation of a sulphur-containing polymer compound of the formula (I), as defined in claim 8, the process comprising reacting a compound of the formula

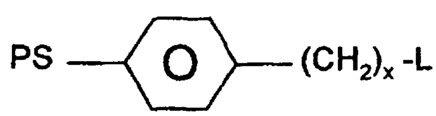

in which PS and x are as defined in claim 8 and L is a leaving group,
with a sulphur-containing nucleophile of the formula

$$R^2\text{-S-}R^1\text{-S-Met}$$

in which $R^1$ and $R^2$ are as defined in claim 8, and Met is an electropositive metal.

**17.** A process for the preparation of a sulphur-containing polymer compound of the formula (IIa) as defined in claim 10, the process comprising reacting together a compound of the formula

$$\text{MetS-}R^4\text{-SMet}$$

in which $R^4$ is as defined in claim 10 and Met is an electropositive metal and a compound of the formula

$$X^1\text{-}R^3\text{-}X^2$$

in which $X^1$, $X^2$ and $R^3$ are as defined in claim 10.

**18.** A process according to claim 17, wherein both of $X^1$ and $X^2$ are halogen.

**19.** A process according to claim 18, wherein both of $X^1$ and $X^2$ are Br.

**20.** Use of a sulphur-containing polymer compound according to any one of claims 8 to 14 as a catalyst in the chlorination of an aromatic compound of formula (A) as defined in claim 1 by a chlorinating agent.

**Patentansprüche**

**1.** Verfahren zur Chlorierung einer aromatischen Verbindung der folgenden Formel (A):

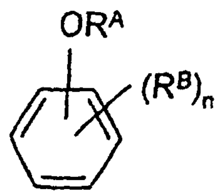

(A)

worin $R^A$ H oder $C_1$-$C_{12}$-Alkyl, Cycloalkyl, Aryl, Alkaryl, Aralkyl oder Carboxyalkyl ist, das oder jedes $R^B$ aus $C_1$-$C_4$-Alkyl, $C_1$-$C_4$-Haloalkyl oder -Polyhaloalkyl, $C_1$-$C_4$-Alkoxy, $C_5$-$C_{12}$-Aryl, -Alkaryl oder -Aralkyl oder Halogen ausgewählt ist, n eine ganze Zahl ist, die 0, 1 oder 2 ist, und das oder jedes $R^B$, falls es vorhanden ist, unabhängig voneinander in ortho- oder meta-Position gebunden sein kann, wobei das Verfahren das Umsetzen der aromatischen Verbindung mit einem Chlorierungsmittel in Gegenwart eines schwefelhältigen Katalysators umfasst, gegebenenfalls auch in Gegenwart eines Lewis-Säure-Co-Katalysators der Formel $MX_a$, worin M ein Metall oder Halbmetall ist; X eine elektronegative Gruppe ist; und m eine ganze Zahl ist;

**dadurch gekennzeichnet, dass** der schwefelhältige Katalysator eine organische Verbindung gemäß nachstehender Formel (I) oder Formel (II) ist:

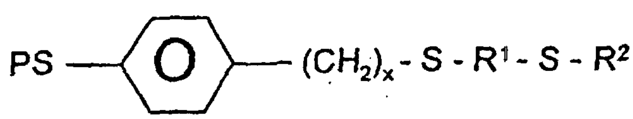

(I)

$$X^1 - R^3 - (S - R^4 - S - R^3)_y - S - R^4 - X^2 \quad \text{(II)}$$

worin:

PS eine Polymerhauptkette darstellt;
x eine ganze Zahl ist, die 0 ist oder 1 bis 12 beträgt;
$R^1$, $R^2$, $R^3$ und $R^4$ jeweils unabhängig voneinander aus der Gruppe bestehend aus gegebenenfalls substituiertem unverzweigtem oder verzweigtem Alkyl oder Alkandiyl mit 1 bis 20 Kohlenstoffatomen und gegebenenfalls substituiertem unverzweigtem oder verzweigtem Alkaryl oder Aralkyl mit 5 bis 20 Kohlenstoffatomen ausgewählt sind;
$X^1$ und $X^2$ jeweils unabhängig voneinander aus der aus Halogen, Thiol, Dialkylsulfoniumhalogenid und $R_5$ bestehenden Gruppe ausgewählt sind, worin $R_5$ aus der aus gegebenenfalls substituiertem, unverzweigtem

oder verzweigtem Alkyl oder Alkandiyl mit 1 bis 20 Kohlenstoffatomen und gegebenenfalls substituiertem, unverzweigtem oder verzweigtem Alkaryl oder Aralkyl mit 5 bis 20 Kohlenstoffatomen bestehenden Gruppe ausgewählt ist;
y eine Zahl (eine ganze Zahl oder keine ganze Zahl) größer als 0 ist;

worin in den obigen Definitionen von $R^1$, $R^2$, $R^3$, $R^4$ und $R^5$ die optionalen Substituenten unabhängig voneinander aus Halogen, Hydroxy, Amino, Cyano, Nitro, $C_1$-$C_4$-Alkyl, $C_1$-$C_4$-Haloalkyl, $C_1$-$C_4$-Alkoxy und ($C_1$-$C_4$-Alkoxy)carbonyl ausgewählt sein können.

**2.** Verfahren nach Anspruch 1, worin die aromatische Verbindung Phenol ist.

**3.** Verfahren nach Anspruch 1, worin die aromatische Verbindung m-Kresol ist.

**4.** Verfahren nach Anspruch 1 oder 2, worin das Chlorierungsmittel Sulfurylchlorid ist.

**5.** Verfahren nach einem der vorhergehenden Ansprüche, worin die Chlorierungsreaktion in heterogener Flüssig/Fest-Phase ohne Zusatz von Lösungsmittel durchgeführt wird.

**6.** Verfahren nach einem der vorhergehenden Ansprüche, worin die Chlorierungsreaktion bei einer Temperatur von unter 35 °C durchgeführt wird.

**7.** Verfahren nach einem der vorhergehenden Ansprüche, worin die Menge an schwefelhältigem Polymer/Harz-Katalysator im Reaktionsgemisch von 0,01 bis 5 g pro 100 mMol der aromatischen Verbindung im Reaktionsgemisch reicht.

**8.** Schwefelhältige Polymerverbindung der Formel:

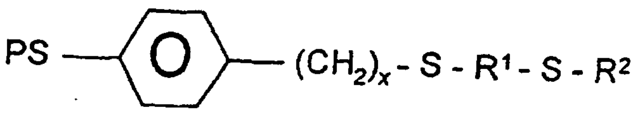

(I)

worin:

PS eine Polymerhauptkette darstellt;
x eine ganze Zahl ist, die 0 ist oder 1 bis 12 beträgt;
$R^1$ und $R^2$ jeweils unabhängig voneinander aus der aus gegebenenfalls substituiertem, unverzweigtem oder verzweigtem Alkyl oder Alkandiyl mit 1 bis 20 Kohlenstoffatomen und gegebenenfalls substituiertem, unverzweigtem oder verzweigtem Alkaryl oder Aralkyl mit 5 bis 20 Kohlenstoffatomen bestehenden Gruppe ausgewählt sind;

worin in den obigen Definitionen von $R^1$ und $R^2$ die optionalen Substituenten unabhängig voneinander aus Halogen, Hydroxy, Amino, Cyano, Nitro, $C_1$-$C_4$-Alkyl, $C_1$-$C_4$-Haloalkyl, $C_1$-$C_4$-Alkoxy und ($C_1$-$C_4$-Alkoxy)carbonyl ausgewählt sein können.

**9.** Verbindung nach Anspruch 8, worin $R^1$ und $R^2$ jeweils unabhängig voneinander aus gegebenenfalls substituiertem, unverzweigtem oder verzweigtem Alkyl oder Alkandiyl mit 1 bis 10 Kohlenstoffatomen ausgewählt sind.

**10.** Schwefelhältige Verbindung der Formel

$$X^1\text{-}R^3\text{-}(S\text{-}R^4\text{-}S\text{-}R^3)_y\text{-}S\text{-}R^4\text{-}X^2 \qquad \text{(IIa)}$$

worin:

$R^3$ und $R^4$ jeweils unabhängig voneinander aus der aus gegebenenfalls substituiertem, unverzweigtem oder verzweigtem Alkyl oder Alkandiyl mit 1 bis 20 Kohlenstoffatomen und gegebenenfalls substituiertem, unverzweigtem oder verzweigtem Alkaryl oder Aralkyl mit 5 bis 20 Kohlenstoffatomen bestehenden Gruppe ausgewählt sind;
$X^1$ und $X^2$ jeweils unabhängig voneinander aus der aus Halogen und Dialkylsulfoniumhalogenid bestehenden Gruppe ausgewählt sind;
y ein Zahl (eine ganze Zahl oder keine ganze Zahl) größer als 0 ist;

worin in den obigen Definitionen von $R^3$ und $R^4$ die optionalen Substituenten unabhängig voneinander aus Halogen, Hydroxy, Amino, Cyano, Nitro, $C_1$-$C_4$-Alkyl, $C_1$-$C_4$-Haloalkyl, $C_1$-$C_4$-Alkoxy und ($C_1$-$C_4$-Alkoxy)carbonyl ausgewählt sein können.

**11.** Verbindung nach Anspruch 10, die erhalten werden kann, indem eine Verbindung der Formel HS-$R^4$-SH mit einem Organolithium-Reagens umgesetzt wird, um eine Verbindung der Formel LiS-$R^4$-SLi zu bilden, und indem anschließend das Produkt mit einer Verbindung der Formel $X^1$-$R^3$-$X^2$ umgesetzt wird, worin $X^1$ und $X^2$ jeweils unabhängig voneinander aus Halogenen ausgewählt sind und $R^3$ und $R^4$ jeweils wie in Anspruch 10 definiert sind.

**12.** Verbindung nach Anspruch 10, worin sowohl $X^1$ als auch $X^2$ Halogen sind.

**13.** Verbindung nach Anspruch 12, worin sowohl $X^1$ als auch $X^2$ Br sind.

**14.** Verbindung nach einem der Ansprüche 10 bis 13, worin $R^3$ und $R^4$ jeweils unabhängig voneinander eine $C_6$-$C_8$-Alkylgruppe sind.

**15.** Verfahren zur Herstellung einer schwefelhältigen Polymerverbindung der Formel (I) wie in Anspruch 8 definiert, wobei das Verfahren das Umsetzen einer Verbindung der Formel:

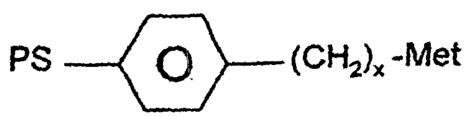

worin PS und x wie in Anspruch 8 definiert sind und Met ein elektropositives Metall ist, mit einer Disulfidverbindung der Formel:

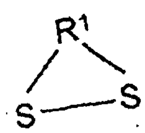

und dann einer Verbindung der Formel L-$R^2$, worin $R^1$ und $R^2$ wie in Anspruch 8 definiert sind und L eine Abgangsgruppe ist, umfasst.

**16.** Verfahren zur Herstellung einer schwefelhältigen Polymerverbindung der Formel (I) wie in Anspruch 8 definiert, wobei das Verfahren das Umsetzen einer Verbindung der Formel:

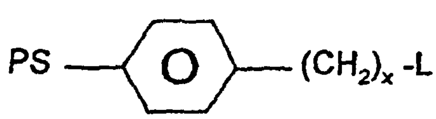

worin PS und x wie in Anspruch 8 definiert sind und Leine Abgangsgruppe ist, mit einem schwefelhältigen Nucleo-

phil der Formel:

$$R^2\text{-S-}R^1\text{-S-Met}$$

worin $R^1$ und $R^2$ wie in Anspruch 8 definiert sind und Met ein elektropositives Metall ist, umfasst.

**17.** Verfahren zur Herstellung einer schwefelhältigen Polymerverbindung der Formel (IIa) wie in Anspruch 10 definiert, wobei das Verfahren das Umsetzen einer Verbindung der Formel:

$$\text{MetS-}R^4\text{-SMet}$$

worin $R^4$ wie in Anspruch 10 definiert ist und Met ein elektropositives Metall ist, und einer Verbindung der Formel:

$$X^1\text{-}R^3\text{-}X^2$$

worin $X^1$, $X^2$ und $R^3$ wie in Anspruch 10 definiert sind, umfasst.

**18.** Verfahren nach Anspruch 17, worin sowohl $X^1$ als auch $X^2$ Halogen sind.

**19.** Verfahren nach Anspruch 18, worin sowohl $X^1$ als auch $X^2$ Br sind.

**20.** Verwendung einer schwefelhältigen Polymerverbindung nach einem der Ansprüche 8 bis 14 als Katalysator bei der Chlorierung einer aromatischen Verbindung der Formel (A) wie in Anspruch 1 definiert mit einem Chlorierungsmittel.

## Revendications

**1.** Procédé pour la chloration d'un composé aromatique de formule (A) suivante :

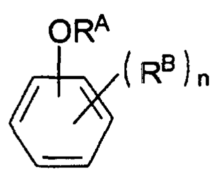

(A)

dans laquelle $R^A$ est H ou un groupe alkyle en $C_1$ à $C_{12}$, un groupe cycloalkyle, aryle, alcaryle, aralkyle ou carboxyalkyle, le ou chaque radical $R^B$ est choisi parmi un groupe alkyle en $C_1$ à $C_4$, un groupe halogénoalkyle en $C_1$ à $C_4$ ou un groupe polyhalogénoalkyle, un groupe alcoxy en $C_1$ à $C_4$, un groupe aryle en $C_5$ à $C_{12}$, un groupe alcaryle ou un groupe aralkyle, ou un halogène, et n est un entier qui vaut 0, 1 ou 2, et le ou chaque radical $R^B$, s'il existe, peut indépendamment être fixé à la position ortho ou méta, ledit procédé comprenant la réaction du composé aromatique avec un agent de chloration en présence d'un catalyseur contenant du soufre, également facultativement en présence d'un co-catalyseur à base d'acide de Lewis de formule $MX_m$, dans laquelle M est un métal ou un métalloïde, X est un groupe électronégatif et m est un entier ; **caractérisé en ce que** le catalyseur contenant du soufre est un composé organique selon la formule (I) ou la formule (II) suivante

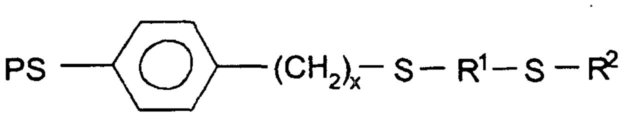

(I)

$$X^1\text{-}R^3\text{-}(S\text{-}R^4\text{-}S\text{-}R^3)_y\text{-}S\text{-}R^4\text{-}X^2 \quad (II)$$

dans laquelle :

PS représente un squelette polymère ;
x est un entier qui vaut 0 ou de 1 à 12 ;
$R^1$, $R^2$, $R^3$ et $R^4$ sont chacun indépendamment choisis dans le groupe comprenant un groupe alkyle ou alcanediyle à chaîne droite ou ramifiée facultativement substitué ayant de 1 à 20 atomes de carbone et un groupe alcaryle ou aralkyle à chaîne droite ou ramifiée facultativement substitué ayant de 5 à 20 atomes de carbone ;
$X^1$ et $X^2$ sont chacun indépendamment choisis dans le groupe comprenant un halogène, un thiol, un halogénure de dialkylsulfonium et $R_5$, où $R_5$ est choisi dans le groupe comprenant un groupe alkyle ou alcanediyle à chaîne droite ou ramifiée facultativement substitué ayant de 1 à 20 atomes de carbone et un groupe alcaryle ou aralkyle à chaîne droite ou ramifiée facultativement substitué ayant de 5 à 20 atomes de carbone ;
y est un nombre (qui peut être entier ou non) supérieur à zéro ;

où dans les définitions ci-dessus de $R^1$, $R^2$, $R^3$, $R^4$ et $R^5$, les substituants facultatifs peuvent être choisis indépendamment parmi les éléments suivants : un halogène, un groupe hydroxy, amino, cyano, nitro, un groupe alkyle en $C_1$ à $C_4$, halogénoalkyle en $C_1$ à $C_4$, alcoxy en $C_1$ à $C_4$ et alcoxy($C_1$-$C_4$)-carbonyle.

2. Procédé selon la revendication 1, dans lequel le composé aromatique est le phénol.

3. Procédé selon la revendication 1, dans lequel le composé aromatique est le métacrésol.

4. Procédé selon la revendication 1 ou la revendication 2, dans lequel l'agent de chloration est le chlorure de sulfuryle.

5. Procédé selon l'une quelconque des revendications précédentes, dans lequel la réaction de chloration est réalisée dans une phase liquide/solide hétérogène, sans présence de solvant ajouté.

6. Procédé selon l'une quelconque des revendications précédentes, dans lequel la réaction de chloration est réalisée à une température au-dessous de 35°C.

7. Procédé selon l'une quelconque des revendications précédentes, dans lequel la quantité de catalyseur polymère contenant du soufre/résine présent dans le mélange réactionnel va de 0,01 g à 5 g pour chaque 100 mmoles du composé aromatique présent dans le mélange réactionnel.

8. Composé polymère contenant du soufre de formule :

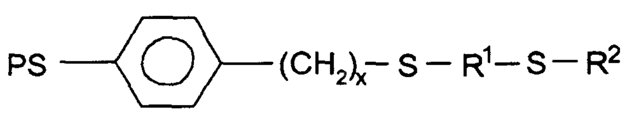

(I)

dans laquelle :

PS est un squelette polymère ;
x est un entier qui vaut 0 ou de 1 à 12 ;
$R^1$ et $R^2$ sont chacun choisis indépendamment dans le groupe comprenant un groupe alkyle ou alcanediyle à chaîne droite ou ramifiée facultativement substitué ayant de 1 à 20 atomes de carbone, et un groupe alcaryle ou aralkyle à chaîne droite ou ramifiée facultativement substitué ayant de 5 à 20 atomes de carbone ;

où, dans les définitions ci-dessus de $R^1$ et de $R^2$, les substituants facultatifs peuvent être indépendamment choisis parmi les éléments suivants : un halogène, un groupe hydroxy, amino, cyano, nitro, un groupe alkyle en $C_1$ à $C_4$, halogénoalkyle en $C_1$ à $C_4$, alcoxy en $C_1$ à $C_4$ et

9. Composé selon la revendication 8, dans lequel $R^1$ et $R^2$ sont chacun indépendamment choisis parmi un groupe

alkyle ou alcanediyle à chaîne droite ou ramifiée facultativement substitué ayant de 1 à 10 atomes de carbone.

**10.** Composé contenant du soufre de formule :

$$X^1\text{-}R^3\text{-}(S\text{-}R^4\text{-}S\text{-}R^3)_y\text{-}S\text{-}R^4\text{-}X^2 \qquad \text{(IIa)}$$

dans laquelle :

$R^3$ et $R^4$ sont chacun indépendamment choisis dans le groupe comprenant un groupe alkyle ou alcanediyle à chaîne droite ou ramifiée facultativement substitué ayant de 1 à 20 atomes de carbone, et un groupe alcaryle ou aralkyle à chaîne droite ou ramifiée facultativement substitué ayant de 5 à 20 atomes de carbone ;
$X^1$ et $X^2$ sont chacun indépendamment choisis dans le groupe comprenant un halogène et un halogénure de dialkylsulfonium ;
y est un nombre (qui peut être entier ou non) supérieur à zéro ;

où dans les définitions ci-dessus de $R^3$ et de $R^4$, les substituants facultatifs peuvent être indépendamment choisis parmi les éléments suivants : un halogène, un groupe hydroxy, amino, cyano, nitro, alkyle en $C_1$ à $C_4$, halogénoalkyle en $C_1$ à $C_4$, alcoxy en $C_1$ à $C_4$ et alcoxy($C_1$-$C_4$)-carbonyle.

**11.** Composé selon la revendication 10, qui peut être obtenu en faisant réagir un composé de formule HS-$R^4$-SH avec un réactif organo-lithium pour former un composé de formule LiS-$R^4$-SLi, puis en faisant réagir le produit avec un composé de formule $X^1$-$R^3$-$X^2$ dans laquelle $X^1$ et $X^2$ sont chacun indépendamment choisis parmi un halogène et chacun des radicaux $R^3$ et $R^4$ est tel que défini dans la revendication 10.

**12.** Composé selon la revendication 10, dans lequel $X^1$ et $X^2$ sont tous deux un halogène.

**13.** Composé selon la revendication 12, dans lequel $X^1$ et $X^2$ sont tous deux Br.

**14.** Composé selon l'une quelconque des revendications 10 à 13, dans lequel $R^3$ et $R^4$ sont chacun indépendamment un groupe alkyle en $C_6$ à $C_8$.

**15.** Procédé pour la préparation d'un polymère contenant du soufre de formule (I), tel que défini dans la revendication 8, le procédé comprenant la réaction d'un composé de formule :

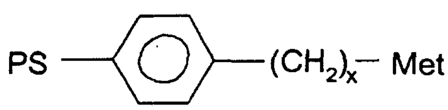

dans laquelle PS et x sont tels que définis dans la revendication 8 et Met est un métal électropositif, avec un composé de disulfure de formule :

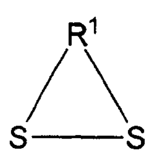

et puis un composé de formule L-$R^2$
dans laquelle $R^1$ et $R^2$ sont tels que définis dans la revendication 8 et L est un groupe partant.

**16.** Procédé pour la préparation d'un composé polymère contenant du soufre de formule (I), tel que défini dans la revendication (8), le procédé comprenant la réaction d'un composé de formule :

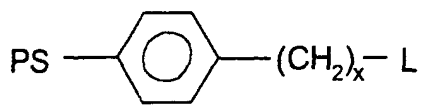

dans laquelle PS et x sont tels que définis dans la revendication 8 et L est un groupe partant, avec le nucléophile contenant du soufre de formule :

$$R^2\text{-S-}R^1\text{-S-Met}$$

dans laquelle $R^1$ et $R^2$ sont tels que définis dans la revendication 8, et Met est un métal électropositif.

**17.** Procédé pour la préparation d'un composé polymère contenant du soufre de formule (IIa) tel que défini dans la revendication 10, le procédé comprenant la réaction conjointe d'un composé de formule :

$$\text{MetS-}R^4\text{-Smet}$$

dans laquelle $R^4$ est tel que défini dans la revendication 10 et Met est un métal électropositif, et d'un composé de formule :

$$X^1\text{-}R^3\text{-}X^2$$

dans laquelle $X^1$, $X^2$ et $R^3$ sont tels que définis dans la revendication 10.

**18.** Procédé selon la revendication 17, dans lequel $X^1$ et $X^2$ sont tous deux un halogène.

**19.** Procédé selon la revendication 18, dans lequel $X^1$ et $X^2$ sont tous deux Br.

**20.** Utilisation d'un composé polymère contenant du soufre selon l'une quelconque des revendications 8 à 14, en tant que catalyseur dans la chloration d'un composé aromatique de formule (A) tel que défini dans la revendication 1 par un agent de chloration.